# EUROPEAN PATENT SPECIFICATION

(11) **EP 4 509 042 B1**
(45) Date of publication and mention of the grant of the patent: **22.04.2026**
(21) Application number: 23864426.4
(22) Date of filing: 28.06.2023
(51) Int. Cl.: A61B 5/022, A61B 5/021

(54) **AIRBAG ASSEMBLY AND WEARABLE DEVICE**
AIRBAGANORDNUNG UND WEARABLE-VORRICHTUNG
ENSEMBLE COUSSIN GONFLABLE ET DISPOSITIF PORTABLE

(30) Priority: 16.09.2022 CN 202211129801
(43) Date of publication of application: 19.02.2025
(73) Proprietor: Huawei Technologies Co., Ltd., Shenzhen, Guangdong 518129 (CN)
(72) Inventor: LI, Ming, Shenzhen, Guangdong 518129 (CN); XUE, Jingtao, Shenzhen, Guangdong 518129 (CN); JIN, Guo Qing, Shenzhen, Guangdong 518129 (CN); CHEN, Qing, Shenzhen, Guangdong 518129 (CN); JIN, Junye, Shenzhen, Guangdong 518129 (CN); WANG, Youhua, Shenzhen, Guangdong 518129 (CN); ZHAI, Yibin, Shenzhen, Guangdong 518129 (CN); SUN, Yufei, Shenzhen, Guangdong 518129 (CN); FENG, Rongkai, Shenzhen, Guangdong 518129 (CN)
(74) Representative: Gill Jennings & Every LLP
(86) International application number: PCT/CN2023/103413
(87) International publication number: WO 2024/055687

(56) References cited:
- CN-A- 107 260 247
- CN-A- 109 717 846
- CN-A- 113 080 904
- CN-A- 114 886 398
- CN-U- 209 826 699
- US-A1- 2010 106 031
- US-A1- 2013 053 708
- US-A1- 2018 042 615

## Description

### TECHNICAL FIELD

This application relates to the field of wearable devices, and in particular, to an airbag assembly and a wearable device.

### BACKGROUND

With popularization of smart wearable devices such as smart watches and smart bands, more functions are integrated into the smart wearable devices. Blood pressure measurement is a highlight characteristic of a new function of the smart wearable device. In a process of blood pressure measurement, an airbag in the wearable device needs to be inflated. After being inflated and expanded, the airbag presses an artery of a user, to measure blood pressure of the user.

Prior art devices are disclosed for example in documents US2010/106031 and US2013/053708.

Currently, a single-layer airbag structure is usually used in the wearable device that performs blood pressure measurement. Because an overall expansion height of the single-layer airbag structure is low, a boundary effect of the single-layer airbag structure is obvious. As a result, it is difficult for a boundary of an airbag to be closely attached to a skin surface of the user. Consequently, it is difficult to provide a sufficient effective pressure area. A double-layer airbag structure makes up for the deficiency of the single-layer airbag structure to some extent, and weakens the boundary effect through superposition of two layers of airbags. However, the double-layer airbag structure has a problem of a high overall expansion height. As a result, the double-layer airbag structure is integrally oversized, has a large thickness, has poor air permeability, is likely to be sweaty, has poor wearing comfort, and has poor user experience. In addition, because the double-layer airbag structure increases a capacity of an inflation cavity of the airbag, actuation efficiency is also reduced to some extent.

The double-layer airbag structure weakens the boundary effect only to some extent, but cannot completely avoid the boundary effect. It is still difficult for a location of the double-layer airbag structure close to a press-fitting part to be closely attached to skin of the user like a middle part, and it is difficult to provide sufficient effective pressure. Therefore, how to weaken the boundary effect and increase the effective pressure area between the airbag and the skin surface of the user while wearing comfort of the user is ensured, to improve accuracy and stability of blood pressure measurement becomes an urgent problem to be resolved.

### SUMMARY

Embodiments of this application provide an airbag assembly and a wearable device, so that an effective pressure area between an airbag and a skin surface of a user is increased through structure design of the airbag assembly, to improve accuracy and stability of blood pressure measurement.

In view of this, embodiments of this application provide the following technical solutions.

According to a first aspect, an embodiment of this application first provides an airbag assembly, and the airbag assembly may be applied to a wearable device for measuring blood pressure. The airbag assembly includes an expansion airbag layer and a protrusion airbag layer disposed on a surface of the expansion airbag layer. A connected first air passage is disposed between the expansion airbag layer and the protrusion airbag layer, and is configured to allow air to circulate between the expansion airbag layer and the protrusion airbag layer. The protrusion airbag layer includes a plurality of sub-airbags. The plurality of sub-airbags include a first sub-airbag and a second sub-airbag. The first sub-airbag is disposed at a location closer to a middle part of the expansion airbag layer than the second sub-airbag. In other words, the second sub-airbag is disposed at a location closer to an edge part (press-fitting part) of the expansion airbag layer than the first sub-airbag. A capacity of an inflation cavity of the first sub-airbag is less than a capacity of an inflation cavity of the second sub-airbag. In other words, a maximum air capacity of the first sub-airbag is less than a maximum air capacity of the second sub-airbag. In other words, in an inflated state, an expansion height of the first sub-airbag is lower than an expansion height of the second sub-airbag.

Usually, an expansion height of the middle part of the expansion airbag layer is high. Due to existence of the boundary effect, the closer to the press-fitting part of the expansion airbag layer, the lower the expansion height of the expansion airbag layer is. Therefore, when the sub-airbag of the protrusion airbag layer is disposed, the capacity of the inflation cavity of the second sub-airbag closer to the press-fitting part of the expansion airbag layer is set to be larger than that of the first sub-airbag, so that the expansion height of the second sub-airbag is higher than that of the first sub-airbag in the inflated state, to better alleviate the boundary effect, increase the expansion height of the part close to the press-fitting part, and increase the effective pressure area, so as to improve accuracy and stability of blood pressure measurement.

In a possible implementation, the expansion airbag layer uses a double-layer airbag structure design, and includes a first expansion airbag layer and a second expansion airbag layer that are stacked. A connected second air passage is disposed between the two expansion airbag layers, and is configured to allow air to circulate between the two expansion airbag layers. The second air passage may be set to a bar-shaped structure. A separation rib is disposed in the second air passage, to divide the second air passage into a plurality of sub-air passages.

Based on the design of the second air passage, an airflow can pass through the air passage more smoothly in an inflation process, so that an expansion effect of the expansion airbag layer is better. In particular, the part close to the press-fitting part can also be fully expanded, to increase the effective pressure area, so as to improve accuracy and stability of blood pressure measurement.

In a possible implementation, the second air passage is disposed in the middle part of the expansion airbag layer, and there is a specific distance between the second air passage and the edge part of the expansion airbag layer. A ratio of a width of the second air passage to a width of the expansion airbag layer is greater than 1:10. To adapt to a shape of a wrist strap of the wearable device, the expansion airbag layer is usually set to a rectangular shape, and includes a long side and a short side. The long side of the expansion airbag layer corresponds to a long side of the wrist strap of the wearable device, and the short side of the expansion airbag layer corresponds to a short side of the wearable device. The width of the expansion airbag layer is determined based on a length of the short side of the expansion airbag layer. A difference between a distance from the second air passage to the long side of the expansion airbag layer and a distance from the second air passage to the short side of the expansion airbag layer is within a preset range. For example, the preset range may be 5 mm. For example, the distance between the second air passage and the long side of the expansion airbag layer and the distance between the second air passage and the short side of the expansion airbag layer may also be set to be the same, and only a specific engineering error exists.

According to the foregoing design, expansion of the expansion airbag layer can be more uniform during inflation, to increase the effective pressure area, so as to improve accuracy and stability of blood pressure measurement.

In a possible implementation, a length of the sub-air passage may be set to be less than 30 mm, so that the expansion airbag layer obtains a better expansion effect in the inflation process. A ratio of the length of the sub-air passage to a length of the separation rib may be further set to be greater than 2 and less than 8, to ensure stability and reliability of the second air passage in the inflation process.

In a possible implementation, the first air passage between the expansion airbag layer and the protrusion airbag layer may be set to a bar-shaped structure. A separation rib is disposed in the first air passage to divide the first air passage into a plurality of sub-air passages.

Based on the design of the first air passage, an airflow can pass through the air passage more smoothly in an inflation process, so that an expansion effect of the protrusion airbag layer is better, to increase the effective pressure area, so as to improve accuracy and stability of blood pressure measurement.

In a possible implementation, a reinforcing rib may be disposed at an appropriate location on an inner wall of the expansion airbag layer or an inner wall of the protrusion airbag layer based on an actual requirement.

Disposing of the reinforcing rib causes a specific suppression effect on expansion of the airbag. Through appropriate design, a shape of the expanded airbag can be further improved, to further increase the effective pressure area, so as to improve accuracy and stability of blood pressure measurement.

In a possible implementation, a reinforcing rib may be disposed at a middle location of the inner wall of the expansion airbag layer or the inner wall of the protrusion airbag layer.

The reinforcing rib is disposed at the middle location of the inner wall of the expansion airbag layer or the inner wall of the protrusion airbag layer, so that expansion of the middle part of the expansion airbag layer or the protrusion airbag layer can be limited to some extent, an expansion height difference between the middle part and the edge part of the airbag assembly can be reduced, to increase the effective pressure area, so as to improve accuracy and stability of blood pressure measurement.

In a possible implementation, a thickness of an airbag wall of the protrusion airbag layer may be set to be less than a thickness of an airbag wall of the expansion airbag layer.

In the foregoing design, in a process of inflating the airbag assembly, the protrusion airbag layer can obtain a better expansion effect than the expansion airbag layer, to increase the effective pressure area, so as to improve accuracy and stability of blood pressure measurement.

In a possible implementation, a thickness of an airbag wall that is of the expansion airbag layer and that is close to the protrusion airbag layer may be set to be less than a thickness of an airbag wall that is of the expansion airbag layer and that is away from the protrusion airbag layer.

The foregoing design can ensure that a side close to the skin surface of the user obtains a better expansion effect, and improve accuracy and stability of blood pressure measurement. In addition, expansion of the airbag away from the skin surface of the user is appropriately limited, so that an overall expansion height of the expanded airbag can be reduced, and wearing comfort can be improved.

In a possible implementation, an airbag wall with an uneven thickness may be disposed for the expansion airbag layer or the protrusion airbag layer.

A thickness of the airbag wall affects expansion of the airbag to some extent. For example, a thick airbag wall imposes a specific limitation on expansion of the airbag to some extent. As a result, in a same airbag, when a location at which the airbag wall is thick is compared with a location at which the airbag wall is thin, an expansion effect of the location at which the airbag wall is thin is better. Therefore, the thickness of the airbag wall is appropriately designed, so that a height difference between an expansion height of a middle part of the airbag assembly and an expansion height of a part close to the press-fitting part can be reduced, to increase the effective pressure area, so as to improve accuracy and stability of blood pressure measurement.

Specifically, in a possible implementation, a thickness of an airbag wall close to the middle part of the expansion airbag layer is greater than a thickness of an airbag wall away from the middle part of the expansion airbag layer. In another possible implementation, a thickness of an airbag wall of the first sub-airbag that is closer to the middle location of the expansion airbag layer compared with the second sub-airbag is greater than a thickness of an airbag wall of the second sub-airbag. In another possible implementation, the plurality of sub-airbags included in the protrusion airbag layer further include a third sub-airbag, and a thickness of an airbag wall close to a middle location of the third sub-airbag is greater than a thickness of an airbag wall away from the middle part of the third sub-airbag.

All the foregoing specific designs of the thickness of the airbag wall can achieve an effect of reducing a height difference between an expansion height of a middle part of the airbag assembly and an expansion height of a part close to the press-fitting part, to increase the effective pressure area, so as to improve accuracy and stability of blood pressure measurement.

In a possible implementation, capacities of the inflation cavities of the plurality of sub-airbags gradually increase in a direction from a location close to the middle part of the expansion airbag layer to a location away from the middle part of the expansion airbag layer. In other words, maximum air capacities of the plurality of sub-airbags gradually increase. In other words, in the inflated state, expansion heights of the plurality of sub-airbags gradually increase.

Based on this gradient design, it can be ensured that a surface height of the expanded airbag assembly is stable, and a better pressure application effect is provided, to improve accuracy and stability of blood pressure measurement.

In a possible implementation, a folding structure may be disposed at a location that is on the airbag wall of the expansion airbag layer and that is close to the press-fitting part. For example, the folding structure may be formed in a hot-pressed airbag wall manner, or the folding structure may be formed in another possible process manner.

The shape of the expanded expansion airbag layer can be further improved by disposing the folding structure.

In a possible implementation, in the plurality of sub-airbags included in the protrusion airbag layer, some sub-airbags are independently disposed (not connected to each other), and some sub-airbags are connected to each other. Specifically, the plurality of sub-airbags include a fourth sub-airbag and a fifth sub-airbag that are adjacent to each other. A connected air passage is disposed between the fourth sub-airbag and the fifth sub-airbag, and is configured to allow air to circulate between the fourth sub-airbag and the fifth sub-airbag. The plurality of sub-airbags further include a sixth airbag adjacent to the fourth sub-airbag. The fourth sub-airbag and the sixth airbag are independent of each other, and no connected air passage is disposed.

A connected air passage is selectively disposed between two adjacent sub-airbags, so that an expansion shape of the inflated protrusion airbag layer can be changed, to adapt to requirements of different users or different wrist strap shapes, and increase the effective pressure area, so as to improve accuracy and stability of blood pressure measurement.

In a possible implementation, the plurality of the sub-airbag included in the protrusion airbag layer may be of a bar-shaped structure, a rectangular structure, a rhombic structure, or a circular structure. The plurality of sub-airbags may be of a same shape, or may be of different shapes.

In a possible implementation, an air inlet is disposed on the expansion airbag layer, and allows communication between an inflation cavity of the expansion airbag layer and extemal air.

According to a second aspect, an embodiment of this application provides an airbag assembly, and the airbag assembly may be applied to a wearable device for measuring blood pressure. The airbag assembly includes an expansion airbag layer. The expansion airbag layer includes a first expansion airbag layer and a second expansion airbag layer that are stacked. A connected second air passage is disposed between the two expansion airbag layers, and is configured to allow air to circulate between the two expansion airbag layers. The second air passage may be set to a bar-shaped structure. A separation rib is disposed in the second air passage, to divide the second air passage into a plurality of sub-air passages.

In a possible implementation, the second air passage is disposed in a middle part of the expansion airbag layer, and there is a specific distance between the second air passage and an edge part of the expansion airbag layer. A ratio of a width of the second air passage to a width of the expansion airbag layer is greater than 1:10. To adapt to a shape of a wrist strap of the wearable device, the expansion airbag layer is usually set to a rectangular shape, and includes a long side and a short side. The long side of the expansion airbag layer corresponds to a long side of the wrist strap of the wearable device, and the short side of the expansion airbag layer corresponds to a short side of the wearable device. The width of the expansion airbag layer is determined based on a length of the short side of the expansion airbag layer. A difference between a distance from the second air passage to the long side of the expansion airbag layer and a distance from the second air passage to the short side of the expansion airbag layer is within a preset range. For example, the preset range may be 5 mm. For example, the distance between the second air passage and the long side of the expansion airbag layer and the distance between the second air passage and the short side of the expansion airbag layer may also be set to be the same, and only a specific engineering error exists.

In a possible implementation, a length of the sub-air passage may be set to be less than 30 mm, so that the expansion airbag layer obtains a better expansion effect in an inflation process. A ratio of the length of the sub-air passage to a length of the separation rib may be further set to be greater than 2 and less than 8, to ensure stability and reliability of the second air passage in the inflation process.

In a possible implementation, the airbag assembly further includes a protrusion airbag layer. A connected first air passage is disposed between the expansion airbag layer and the protrusion airbag layer, and is configured to allow air to circulate between the expansion airbag layer and the protrusion airbag layer. The protrusion airbag layer includes a plurality of sub-airbags. The plurality of sub-airbags include a first sub-airbag and a second sub-airbag. The first sub-airbag is disposed at a location closer to the middle part of the expansion airbag layer than the second sub-airbag. In other words, the second sub-airbag is disposed at a location closer to the edge part (press-fitting part) of the expansion airbag layer than the first sub-airbag. A capacity of an inflation cavity of the first sub-airbag is less than a capacity of an inflation cavity of the second sub-airbag. In other words, a maximum air capacity of the first sub-airbag is less than a maximum air capacity of the second sub-airbag. In other words, in an inflated state, an expansion height of the first sub-airbag is lower than an expansion height of the second sub-airbag.

According to a third aspect, an embodiment of this application provides a wearable device, and the wearable device may be configured to perform blood pressure measurement. The wearable device includes a watch body, a pressure sensor, and the airbag assembly described in the first aspect or the second aspect. The pressure sensor is electrically connected to the watch body, and is configured to detect air pressure in the airbag assembly.

In a possible implementation, the wearable device further includes a wrist strap, and the airbag assembly is disposed in the wrist strap.

The second aspect and the third aspect of the embodiments of this application can implement the beneficial effects described in the first aspect. To avoid repetition, details are not described herein again.

### BRIEF DESCRIPTION OF DRAWINGS

FIG. 1 is a diagram of a structure of a single-layer airbag assembly according to an embodiment of this application;
FIG. 2 is a diagram of a structure of a double-layer airbag assembly according to an embodiment of this application;
FIG. 3 is a top view of a single-layer airbag assembly according to an embodiment of this application;
FIG. 4 is a side view of an expanded single-layer airbag according to an embodiment of this application;
FIG. 5 is a diagram of an effective pressure area of an airbag assembly according to an embodiment of this application;
FIG. 6 is a diagram of a structure of an airbag assembly according to an embodiment of this application;
FIG. 7 is a diagram of a structure of an expansion airbag layer according to an embodiment of this application;
FIG. 8 is a diagram of a structure of a protrusion airbag layer according to an embodiment of this application;
FIG. 9 is a diagram of a structure of another protrusion airbag layer according to an embodiment of this application;
FIG. 10 is a diagram of a structure of still another protrusion airbag layer according to an embodiment of this application;
FIG. 11 is a diagram of a structure of another airbag assembly according to an embodiment of this application;
FIG. 12 is a diagram of a structure of still another airbag assembly according to an embodiment of this application;
FIG. 13 is a diagram of a structure of an air passage according to an embodiment of this application;
FIG. 14 is a diagram of a structure of another air passage according to an embodiment of this application;
FIG. 15a is a diagram of a structure of yet another airbag assembly according to an embodiment of this application;
FIG. 15b is a diagram of a structure of still yet another airbag assembly according to an embodiment of this application;
FIG. 15c is a diagram of a structure of a further airbag assembly according to an embodiment of this application;
FIG. 16a is a diagram of a structure of a still further airbag assembly according to an embodiment of this application;
FIG. 16b is a diagram of a structure of a yet further airbag assembly according to an embodiment of this application;
FIG. 17 is a diagram of a structure of a still yet further airbag assembly according to an embodiment of this application;
FIG. 18 is a diagram of a structure of another expansion airbag layer according to an embodiment of this application; and
FIG. 19 is a diagram of a structure of a wearable device according to an embodiment of this application.

### Reference numerals:

1: single-layer airbag structure; 101: middle part of a single-layer airbag; 102: press-fitting part of a single-layer airbag; 2: double-layer airbag structure; 201: middle part of a double-layer airbag structure; 202: press-fitting part of a double-layer airbag structure; 301: air inlet; 302: press-fitting part on a boundary of an airbag; 6: airbag assembly; 601: expansion airbag layer; 602: protrusion airbag layer; 603: first air passage; 6021: first sub-airbag; 6022: second sub-airbag; 6011: airbag wall of an expansion airbag layer; 6012: air inlet; 6013: inflation cavity; 6014: first expansion airbag layer; 6015: second expansion airbag layer; 604: second airbag; 6042: sub-air passage; 6041: separation rib; 6016: reinforcing rib on an inner wall of an expansion airbag layer; 6023: reinforcing rib on an inner wall of a protrusion airbag layer; 60111: first airbag wall; 60112: second airbag wall; 7: watch body.

### DESCRIPTION OF EMBODIMENTS

The following describes embodiments of the present invention with reference to the accompanying drawings in embodiments of the present invention. In the following descriptions, specific aspects of embodiments of the present invention or accompanying drawings in which specific aspects of embodiments of the present invention may be used are shown. It should be understood that embodiments of the present invention may be used in other aspects, and may include structural or logical changes not depicted in the accompanying drawings. Therefore, the following detailed description shall not be taken in a limiting sense, and the scope of the present invention is defined by the appended claims. For example, it should be understood that disclosed content in combination with a described method may also hold true for a corresponding device or system configured to perform the method and vice versa. For example, if one or more specific method steps are described, a corresponding device may include one or more units such as functional units to perform the described one or more method steps (for example, one unit performing the one or more steps, or a plurality of units each performing one or more of the plurality of steps), even if such one or more units are not explicitly described or illustrated in the accompanying drawings. In addition, for example, if a specific apparatus is described based on one or more units such as functional units, a corresponding method may include a step used to perform functionality of the one or more units (for example, one step used to perform the functionality of the one or more units, or a plurality of steps each used to perform functionality of one or more of a plurality of units), even if such one or more steps are not explicitly described or illustrated in the accompanying drawings. Further, it should be understood that features of example embodiments and/or aspects described herein may be combined with each other, unless expressly stated otherwise.

In embodiments of the present invention, "at least one" means one or more, and "a plurality of" means two or more. The term "and/or" describes an association relationship between associated objects and may indicate three relationships. For example, A and/or B may indicate the following cases: Only A exists, both A and B exist, and only B exists. A and B may be singular or plural. The character "/" usually indicates an "or" relationship between the associated objects. "At least one of the following items (pieces)" or a similar expression thereof means any combination of these items, including any combination of singular items (pieces) or plural items (pieces). For example, at least one item (piece) of a, b, or c may indicate: a, b, c, a and b, a and c, b and c, or a, b, and c, where a, b, and c may be singular or plural.

Terms used in the implementation part of the present invention are merely intended to explain specific embodiments of the present invention, but are not intended to limit the present invention.

With popularization of smart wearable devices such as watches and bands, more functions are integrated into the smart wearable devices. Blood pressure measurement is a highlight characteristic of a new function of the smart wearable device. In a process of blood pressure measurement, an airbag in the wearable device needs to be inflated. After being inflated and expanded, the airbag presses an artery of a user through pressure application, to measure blood pressure of the user.

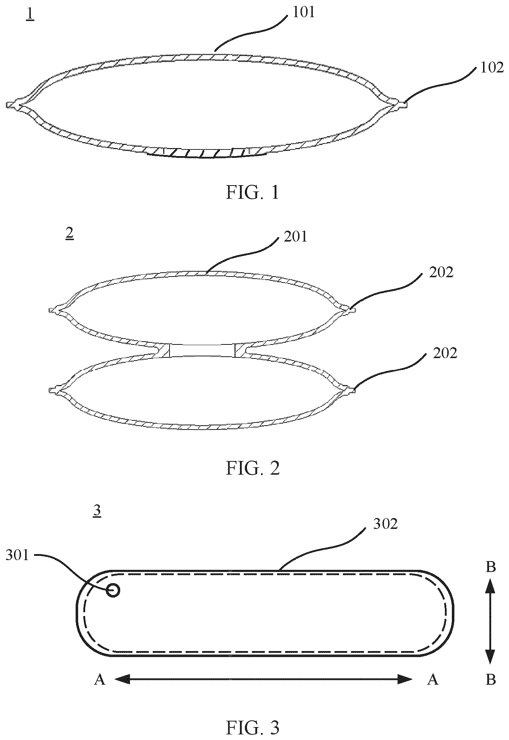

Currently, a single-layer airbag structure is usually used in the wearable device that performs blood pressure measurement. FIG. 1 is a side view of an inflated single-layer airbag structure 1. It can be learned that a shape similar to an ellipse is formed on a side section of the inflated single-layer airbag structure. An expansion height of a middle part 101 is high, and a good contact may be formed with a skin surface of a user to provide effective pressure. However, because an expansion height of a part close to a press-fitting part 102 is far lower than that of the middle part, it is difficult to form a good contact with the skin surface of the user, and it is difficult to provide effective pressure.

FIG. 2 is a side view of an inflated double-layer airbag structure 2. The double-layer airbag structure is an improvement on the single-layer airbag structure, so that a boundary effect is weakened and an effective pressure area is increased. The double-layer airbag structure includes two layers of single-layer airbags disposed in parallel. Compared with the single-layer airbag, an expansion height of a middle part 201 increases, and an expansion height of a part close to a press-fitting part 202 also increases. The double-layer airbag structure makes up for the deficiency of the single-layer airbag structure to some extent, and weakens the boundary effect through superposition of two layers of airbags. However, a design of the double-layer airbag structure also has a plurality of problems: 1. An overall expansion height is high, so that the double-layer airbag structure is integrally oversized, has a large thickness, has poor air permeability, is easy to be sweaty, has poor wearing comfort, and has poor user experience. 2. Because the double-layer airbag structure increases a capacity of an inflation cavity of the airbag, air demand increases during inflating, and actuation efficiency is also reduced to some extent. 3. The double-layer airbag structure weakens the boundary effect only to some extent, but cannot completely avoid the boundary effect. It is still difficult for a boundary of the double-layer airbag structure to be closely attached to skin of the user as a middle location, and it is difficult to provide sufficient effective pressure.

It can be learned that both the single-layer airbag structure and the double-layer airbag structure cannot well resolve the problem of the boundary effect, and it is difficult to provide sufficient effective pressure when the single-layer airbag structure and the double-layer airbag structure are applied to the wearable device. As a result, accuracy and stability of blood pressure measurement in a use process cannot be ensured. Therefore, how to weaken the boundary effect and increase the effective pressure area between the airbag and the skin surface of the user while wearing comfort of the user is ensured, to improve accuracy and stability of blood pressure measurement becomes an urgent problem to be resolved.

A technical problem to be resolved in embodiments of this application is described in detail by using a single-layer airbag structure as an example. As shown in FIG. 3, an airbag in a wearable device is usually of an approximate rectangular structure, and is configured to adapt to a shape of a wrist strap of the wearable device. In FIG. 3, a direction of A-A is a direction of a length of the wrist strap, and a direction of B-B is a direction of a width of the wrist strap. An airbag structure 3 includes at least one air inlet 301, configured to inflate the airbag. To implement sealing of the airbag, press-fitting processing is performed on a boundary of the airbag. Therefore, the airbag structure 3 further includes a press-fitting part 302 located on the boundary of the airbag.

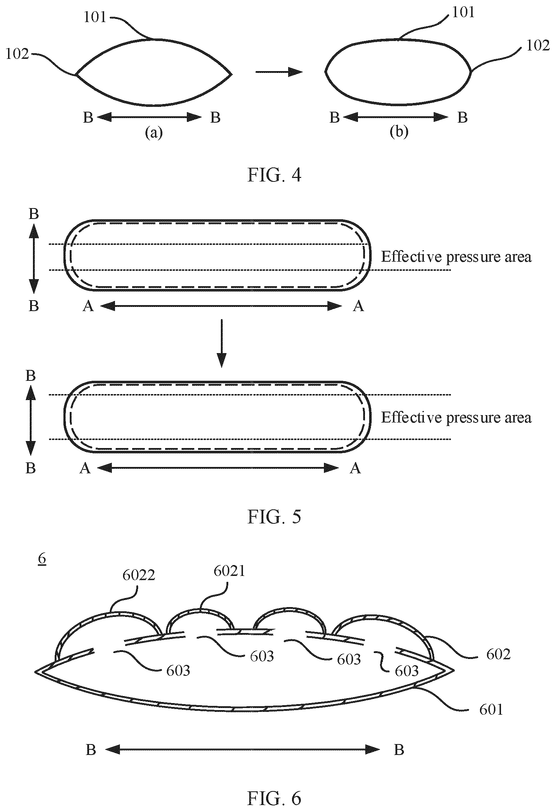

As shown in FIG. 4, (a) is a side view of an inflated common single-layer airbag structure. It can be seen that, affected by a press-fitting part 102, a part close to the press-fitting part has a poor expansion effect. As a result, an expansion height from a middle part 101 of the airbag to the part close to the press-fitting part 102 gradually decreases. In this case, the middle part 101 of the airbag may be closely attached to a skin surface of a user, to achieve a good pressure application effect. The pressure application effect gradually deteriorates from the middle part 101 of the airbag to the press-fitting part 102. As shown in FIG. 4, (a) is a top view of an inflated common single-layer airbag structure. The middle part may provide effective pressure, but the part close to the press-fitting part cannot provide effective pressure. In the top view shown in FIG. 4, an area that can provide effective pressure in the airbag structure is referred to as an effective pressure area.

To resolve the foregoing problem, in the conventional technology, a method of increasing a capacity of an inflation cavity of an airbag is usually used to increase an overall expansion height of the airbag and further increase an effective pressure area. However, this method causes a high overall expansion height of the airbag, so that a wrist strap of a wearable device is thick. In another method, a width of an airbag is increased to increase an effective pressure area. However, in this manner, the width of the airbag is large, and a wrist strap of a wearable device is wide. Both the two methods affect aesthetics of the wearable device and wearing comfort of the user to some extent.

Therefore, as shown in FIG. 4, a main problem to be resolved in embodiments of this application is how to increase an effective pressure area by improving a design of an airbag, so that a side view of an expanded airbag is shown in (b) in FIG. 4. Compared with the expanded common airbag (a), attenuation of an expansion height the expanded airbag (b) from the middle part 101 to the part close to the press-fitting part 102 is small, so that the expanded airbag (b) can have a larger effective pressure area with the skin surface of the user, and a better pressure application effect is provided.

As shown in FIG. 5, a design of an airbag is improved. On one hand, an effective pressure area can be increased while a width of the airbag remains unchanged, accuracy and stability of blood pressure measurement can be improved, and a measurement error caused by an incorrect wearing method or different wrist thickness can be effectively improved. On the other hand, an expansion height of a middle part of the airbag can be reduced while a same effective pressure area is maintained, so that the wrist strap of the wearable device is lighter and thinner, and wearing comfort of the user is improved. A width of the airbag may be further reduced, so that the wrist strap of the wearable device is narrower, and wearing comfort of the user is improved. A volume of inflated air required for airbag expansion may be further reduced, so that actuation efficiency of inflating the airbag is improved.

For the foregoing boundary effect problem existing in a conventional airbag structure, an embodiment of this application provides an airbag assembly that can provide a large effective pressure area during expansion. For details, refer to FIG. 6. FIG. 6 is a diagram of a structure of an airbag assembly according to an embodiment of this application. Specifically, FIG. 6 is a side view of the airbag assembly according to this embodiment of this application. When the airbag assembly shown in FIG. 6 is used in a smart wearable device to measure blood pressure, a direction of B-B in the figure corresponds to a width direction of a wrist strap. As shown in FIG. 6, the airbag assembly provided in this embodiment of this application includes an expansion airbag layer 601 and a protrusion airbag layer 602. The protrusion airbag layer 602 is disposed on a surface of the expansion airbag layer 601. A connected first air passage 603 is disposed between the protrusion airbag layer 602 and the expansion airbag layer 601, and is configured to allow air to circulate between the expansion airbag layer 601 and the protrusion airbag layer 602. When the airbag assembly shown in FIG. 6 is used in the smart wearable device to perform blood pressure measurement, the protrusion airbag layer 602 is a side close to a skin surface of a user, and the expansion airbag layer is a side away from the skin surface of the user.

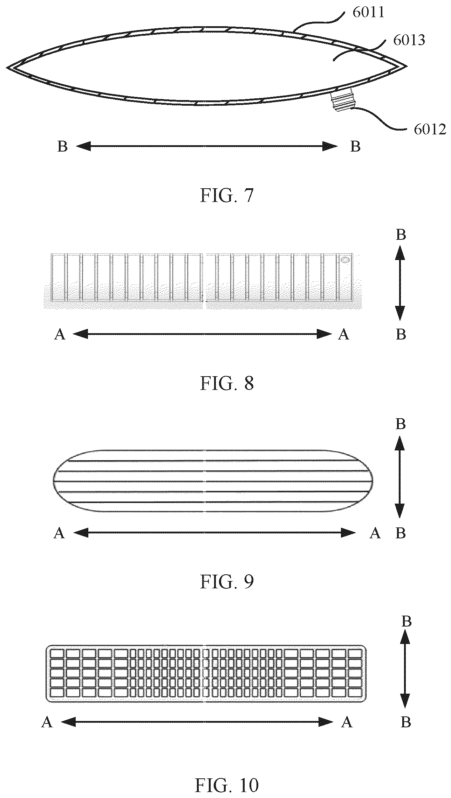

The expansion airbag layer 601 is configured to perform an expansion function, to ensure that a specific expansion height can be formed after the entire airbag assembly is inflated, so as to provide sufficient pressure. As shown in FIG. 7, the expansion airbag layer 601 includes an airbag wall 6011, an air inlet 6012, and an inflation cavity 6013. The air inlet 6012 is disposed on the expansion airbag layer 601. The air inlet 6012 allows communication between the inflation cavity 6013 of the expansion airbag layer 601 and external air, and is configured to inflate external air into the inflation cavity, or exhaust air in the inflation cavity. It should be understood that the air inlet 6012 may be disposed at any location of the expansion airbag layer based on an actual requirement. This is not limited in embodiments of this application.

In another possible implementation, air inlets may alternatively be separately disposed on the expansion airbag layer 601 and the protrusion airbag layer 602, and inflation is performed at the same time, to improve inflation efficiency and reduce inflation time. In this design, the first air passage 603 may be disposed between the expansion airbag layer 601 and the protrusion airbag layer 602, or the first air passage 603 may not be disposed.

The protrusion airbag layer includes a plurality of protrusion sub-airbags, and the sub-airbag includes an airbag wall and an inflation cavity. A quantity and sizes of sub-airbags may be designed based on an actual situation. This is not limited in embodiments of this application. Similarly, a specific shape of the sub-airbag is not limited in embodiments of this application. For example, the sub-airbag may be of a bar-shaped structure shown in FIG. 8 or FIG. 9, or the sub-airbag may be of a rectangular structure shown in FIG. 10. Alternatively, the sub-airbag may be of a rhombic structure, a circular structure, or the like, or may be of any other possible shape. Shapes of the sub-airbags may be the same or may be different. This is not limited in this application.

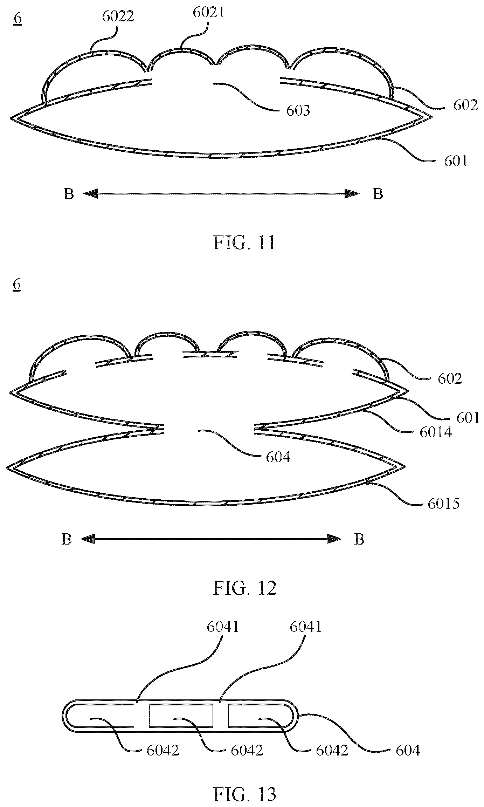

In a possible implementation, as shown in FIG. 6, the protrusion airbag layer 602 includes a plurality of sub-airbags disposed independently of each other. In this case, inflation cavities of the sub-airbags are not connected to each other. A connected air passage 603 is disposed between each sub-airbag and the expansion airbag layer 601, and is configured to allow air to circulate between the expansion airbag layer 601 and each sub-airbag. In another possible implementation, as shown in FIG. 11, the plurality of sub-airbags included in the protrusion airbag layer are connected to each other, that is, inflation cavities of the sub-airbags are connected to each other. In addition, the connected air passage 603 is disposed between the protrusion airbag layer 602 and the expansion airbag layer 601, and is configured to allow air to circulate between the expansion airbag layer 601 and the protrusion airbag layer 602. One or more air passages 603 may be disposed. In another possible implementation, in the plurality of sub-airbags included in the protrusion airbag layer 602, some sub-airbags may be set to be connected, and some sub-airbags may be set to be independent. A specific setting of connection or independence between the sub-airbags may be designed based on an actual situation. This is not limited in embodiments of this application.

In a possible implementation, in the plurality of sub-airbags included in the protrusion airbag layer 602, some sub-airbags are independently disposed (not connected to each other), and some sub-airbags are connected to each other. Specifically, the plurality of sub-airbags include a fourth sub-airbag and a fifth sub-airbag that are adjacent to each other. A connected air passage is disposed between the fourth sub-airbag and the fifth sub-airbag, and is configured to allow air to circulate between the fourth sub-airbag and the fifth sub-airbag. The plurality of sub-airbags further include a sixth airbag adjacent to the fourth sub-airbag. The fourth sub-airbag and the sixth airbag are independent of each other, and no connected air passage is disposed.

A connected air passage is selectively disposed between two adjacent sub-airbags, so that an expansion shape of the inflated protrusion airbag layer can be changed, to adapt to requirements of different users or different wrist strap shapes, and increase the effective pressure area, so as to improve accuracy and stability of blood pressure measurement.

The protrusion airbag layer including the plurality of sub-airbags is disposed on the surface of the expansion airbag layer, so that boundary effect caused by a press-fitting part can be alleviated to some extent, an expansion height of a part close to the press-fitting part can be increased, to increase the effective pressure area, so as to improve accuracy and stability of blood pressure measurement.

In a possible implementation, as shown in FIG. 6, the plurality of sub-airbags include a first sub-airbag 6021 and a second sub-airbag 6022. The first sub-airbag 6021 is disposed at a location closer to a middle part of the expansion airbag layer 601 than the second sub-airbag 6022. In other words, the first sub-airbag 6021 is disposed at a location farther away from the press-fitting part of the expansion airbag layer 601 than the second sub-airbag 6022. A capacity of an inflation cavity of the first sub-airbag 6021 is less than a capacity of an inflation cavity of the second sub-airbag 6022. In other words, a maximum air capacity of the first sub-airbag 6021 is less than a maximum air capacity of the second sub-airbag 6022. In other words, in an inflated state, an expansion height of the first sub-airbag 6021 is less than an expansion height of the second sub-airbag 6022.

Usually, as described above, an expansion height of the middle part of the expansion airbag layer 601 is high. Due to existence of the boundary effect, the closer to the press-fitting part of the expansion airbag layer 601, the lower the expansion height of the expansion airbag layer 601 is. Therefore, when the sub-airbag of the protrusion airbag layer 602 is disposed, the capacity of the inflation cavity of the second sub-airbag 6022 closer to the press-fitting part of the expansion airbag layer 601 is set to be larger than that of the first sub-airbag 6021, so that the expansion height of the second sub-airbag 6022 is higher than that of the first sub-airbag 6021 in the inflated state, to better alleviate the boundary effect, increase the expansion height of the part close to the press-fitting part, and increase the effective pressure area, so as to improve accuracy and stability of blood pressure measurement.

In a possible implementation, capacities of the inflation cavities of the plurality of sub-airbags gradually increase in a direction from a location close to the middle part of the expansion airbag layer 601 to a location away from the middle part of the expansion airbag layer. In other words, maximum air capacities of the plurality of sub-airbags gradually increase. In other words, in the inflated state, expansion heights of the plurality of sub-airbags gradually increase.

Based on this gradient design, it can be ensured that a surface height of the expanded airbag assembly is stable, and a better pressure application effect is provided, to improve accuracy and stability of blood pressure measurement.

In a possible implementation, the expansion airbag layer 601 includes only a single-layer expansion airbag. In another possible implementation, as shown in FIG. 12, the expansion airbag layer 601 includes a first expansion airbag layer 6014 and a second expansion airbag layer 6015 that are stacked. A connected second air passage 604 is disposed between the first expansion airbag layer 6014 and the second expansion airbag layer 6015, and is configured to allow air to circulate between the first expansion airbag layer 6014 and the second expansion airbag layer 6015. An overall expansion height of the airbag assembly can be increased by increasing a quantity of expansion airbag layers. In another possible implementation, the expansion airbag layer 601 may further include more stacked expansion airbag layers, for example, a third expansion airbag layer and a fourth expansion airbag layer. The quantity of expansion airbag layers is not limited in embodiments of this application, and may be designed based on an actual situation.

In a possible implementation, the air inlet 6012 may be disposed on any expansion airbag layer based on a requirement, and an air passage connected between the expansion airbag layers is used to indirectly inflate an airbag layer on which no air inlet is disposed. In another possible implementation, one air inlet may be disposed for each expansion airbag layer. In this case, the second air passage 604 may be disposed between the first expansion airbag layer and the second expansion airbag layer, or the second air passage 604 may not be disposed. In another possible implementation, an air inlet may be disposed for several expansion airbag layers. A connected air passage between the expansion airbag layers is used to inflate another expansion airbag layer on which no air inlet is disposed. A specific manner of disposing the air inlet is not limited in embodiments of this application.

In a possible implementation, as shown in FIG. 13, the second air passage 604 is of a bar-shaped structure. Separation ribs 6041 are disposed in the second air passage 604, and the separation ribs 6041 divide the second air passage into a plurality of sub-air passages 6042.

When the air passage structure shown in FIG. 13 is used to inflate the airbag assembly, an airflow can pass through the air passage more smoothly, so that an expansion effect of the expansion airbag layer is better. In particular, the part close to the press-fitting part can also be fully expanded, to increase the effective pressure area, so as to improve accuracy and stability of blood pressure measurement.

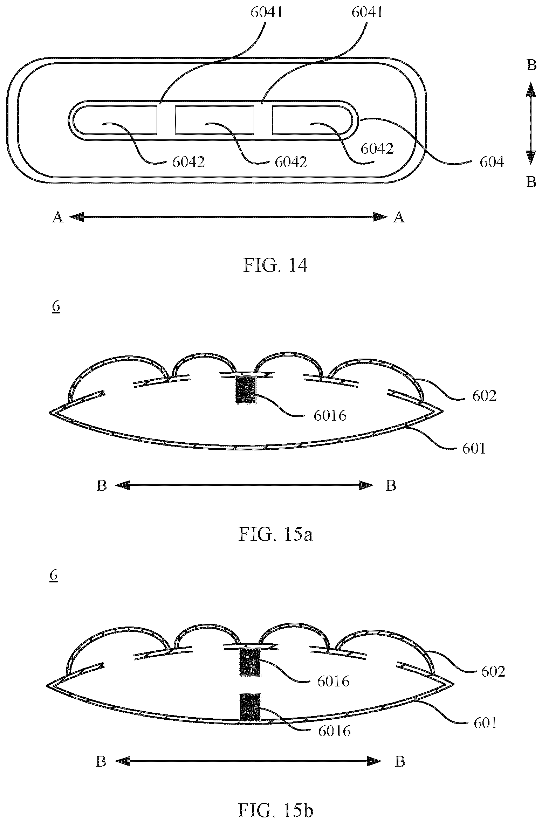

In a possible implementation, as shown in FIG. 14, the second air passage 604 may be disposed in the middle part of the expansion airbag layer 601, and there is a preset distance between the second air passage 604 and a periphery of the expansion airbag layer 601. In FIG. 14, A-A is a direction of a long side of the expansion airbag layer 601. The direction of the long side of the expansion airbag layer 601 corresponds to a length direction of the wrist strap of the wearable device. A length of the long side of the expansion airbag layer 601 is referred to as a length of the expansion airbag layer. B-B is a direction of a short side of the expansion airbag layer 601. The direction of the short side of the expansion airbag layer 601 corresponds to the width direction of the wrist strap of the wearable device. A length of the short side of the expansion airbag layer 601 is referred to as a width of the expansion airbag layer. In a possible implementation, a ratio of a width of the second air passage 604 to the width of the expansion airbag layer 601 is greater than 1:10. In addition, a difference between a distance from the second air passage 604 to the long side of the expansion airbag layer and a distance from the second air passage 604 to the short side of the expansion airbag layer is within a preset range. For example, the preset range may be 5 mm. For example, the distance between the second air passage 604 and the long side of the expansion airbag layer and the distance between the second air passage 604 and the short side of the expansion airbag layer may also be set to be the same, and only a specific engineering error exists.

According to the foregoing design, expansion of the expansion airbag layer 601 can be more uniform during inflation, to increase the effective pressure area, so as to improve accuracy and stability of blood pressure measurement.

For a specific design of the sub-air passage 6042 and the separation rib 6041 in the second air passage 604, in a possible implementation, a length of the sub-air passage 6042 may be set to be less than 30 mm, so that when the expansion airbag layer is inflated through the sub-air passage, and the expansion effect of the expansion airbag layer is better. In addition, to ensure that the separation rib 6041 can stably divide the second airbag 604 into the plurality of sub-air passages 6042, and ensure reliability of the second airbag 604 in an inflation process, a ratio of the length of the sub-air passage 6042 to the length of the separation rib 6041 may be set to be greater than 2 and less than 8. On this basis, the length of the separation rib can be set to 4 mm to 10 mm.

In a possible implementation, the first air passage 603 between the expansion airbag layer 601 and the protrusion airbag layer 602 may also be disposed in the structure shown in FIG. 13. In other words, the first air passage 603 is of a bar-shaped structure. Separation ribs are disposed in the first air passage 603, and the separation ribs divide the first air passage 603 into a plurality of sub-air passages. When the design shown in FIG. 6 is used, the first airbag 603 may be disposed in a middle part of the sub-airbag, and there is a preset distance between the first airbag 603 and an edge part of the sub-airbag (a part at which the airbag wall of the sub-airbag and the airbag wall of the expansion airbag layer are press-fitted). When the design shown in FIG. 11 is used, the first airbag 603 may be disposed in a middle part of the protrusion airbag layer 602, and there is a preset distance between the first airbag 603 and an edge part of the protrusion airbag layer 602 (a part at which an airbag wall of the protrusion airbag layer and the airbag wall of the expansion airbag layer are press-fitted).

To further improve a shape of the expanded airbag, a reinforcing rib 6016 may be disposed at an appropriate location on an inner wall of the expansion airbag layer 601 based on an actual requirement. Disposing of the reinforcing rib 6016 causes a specific suppression effect on expansion of the airbag, to change the shape of the expanded airbag. The reinforcing rib 6016 may be disposed in various shapes, in different sizes, and at different locations based on an actual requirement. Shapes, sizes, a quantity, locations, and the like of the reinforcing ribs 6016 are not limited in embodiments of this application.

Through appropriate design of the location of the reinforcing rib 6016, the effective pressure area can be further increased, to improve accuracy and stability of blood pressure measurement.

In a possible implementation, the reinforcing rib 6016 may be disposed at a middle location of the inner wall of the expansion airbag layer 601. For example, as shown in FIG. 15a, the reinforcing rib 6016 may be disposed only on an inner wall of an airbag wall close to the protrusion airbag layer 602. As shown in FIG. 15b, reinforcing ribs 6016 may alternatively be oppositely disposed on inner walls of two airbag walls. The reinforcing rib 6016 is disposed at the middle location of the inner wall of the expansion airbag layer 601, so that expansion of the middle part of the expansion airbag layer 601 can be limited to some extent, an expansion height difference between the middle part of the expansion airbag layer 601 and the part close to the press-fitting part can be reduced, to increase the effective pressure area, so as to improve accuracy and stability of blood pressure measurement.

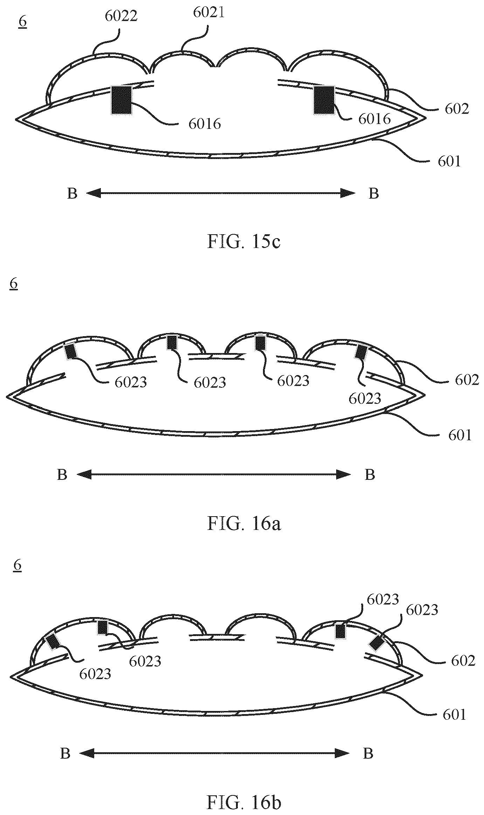

In another possible implementation, the reinforcing rib 6016 may alternatively be disposed at another location on an inner side of the airbag wall of the expansion airbag layer 601. For example, the reinforcing rib 6016 may be disposed at a location shown in FIG. 15c.

With reference to disposition of the reinforcing rib 6016 on the inner wall of the expansion airbag layer 601, a reinforcing rib 6023 may be disposed at an appropriate location on an inner wall of the protrusion airbag layer 602 based on an actual requirement. The reinforcing rib 6023 may cause a specific suppression effect on expansion of the airbag, to change the shape of the expanded airbag. The reinforcing rib 6023 may be disposed in various shapes, in different sizes, and at different locations based on an actual requirement. Shapes, sizes, a quantity, locations, and the like of the reinforcing ribs 6023 are not limited in embodiments of this application.

Through appropriate design of the location of the reinforcing rib 6023, the effective pressure area can be further increased, to improve accuracy and stability of blood pressure measurement.

In a possible implementation, the reinforcing rib 6023 may be disposed at a middle location inside the protrusion airbag layer 602. For example, as shown in FIG. 16a, the reinforcing rib 6023 may be disposed in a middle part of an inner wall of the sub-airbag of the protrusion airbag layer. The reinforcing rib 6023 is disposed at the middle location on the inner wall of the sub-airbag, so that expansion of the middle part of the sub-airbag can be limited to some extent, an expansion height difference between the middle part and an edge part of the sub-airbag can be reduced, to increase the effective pressure area, so as to improve accuracy and stability of blood pressure measurement.

In another possible implementation, the reinforcing rib 6023 may alternatively be disposed at another location on an inner side of the airbag wall of the sub-airbag. For example, the reinforcing rib 6023 may be disposed at a location shown in FIG. 16b.

A thickness of the airbag wall affects expansion of the airbag to some extent. For example, a thick airbag wall imposes a specific limitation on expansion of the airbag to some extent. As a result, in a same airbag, when a location at which the airbag wall is thick is compared with a location at which the airbag wall is thin, an expansion effect of the location at which the airbag wall is thin is better. Based on this, to further improve the shape of the expanded airbag, the thickness of the airbag wall of the airbag assembly may be designed based on an actual requirement.

In a possible implementation, an airbag wall with an uneven thickness may be set for the expansion airbag layer 601 or the protrusion airbag layer 602. For example, a thickness of an airbag wall of a part at which expansion suppression is expected (a lower expansion height) may be designed to be thicker, and a thickness of an airbag wall of a part at which a better expansion effect is expected (a higher expansion height) may be designed to be thinner. For example, a thickness of an airbag wall close to the middle part of the expansion airbag layer 601 may be set to be greater than a thickness of an airbag wall close to the press-fitting part of the expansion airbag layer 601; and/or a thickness of an airbag wall of the first sub-airbag close to the middle part of the expansion airbag layer 601 may be set to be greater than a thickness of an airbag wall of the second sub-airbag away from the middle part of the expansion airbag layer 602; and/or a thickness of the airbag wall of the sub-airbag of the protrusion airbag layer 602 may be designed as that a thickness of an airbag wall close to a middle part is greater than a thickness of an airbag wall away from the middle part of the sub-airbag.

In a possible implementation, a thickness of an airbag wall of the protrusion airbag layer 602 may be set to be less than a thickness of an airbag wall of the expansion airbag layer 601. In this way, in a process of inflating the airbag assembly, the protrusion airbag layer can obtain a better expansion effect than the expansion airbag layer, to increase the effective pressure area, so as to improve accuracy and stability of blood pressure measurement.

In a possible implementation, a thickness of an airbag wall that is of the expansion airbag layer 601 and that is close to the protrusion airbag layer 602 may be set to be less than a thickness of an airbag wall that is of the expansion airbag layer 601 and that is away from the protrusion airbag layer 602. When an airbag assembly 6 is applied to the wrist strap of the wearable device, the protrusion airbag layer is a layer close to the skin surface of the user, and the expansion airbag layer is a side away from the skin surface. Based on the foregoing design, it can be ensured that the side close to the skin surface of the user obtains a better expansion effect, to improve accuracy and stability of blood pressure measurement. In addition, expansion of the airbag away from the skin surface of the user is appropriately limited, so that an overall expansion height of the expanded airbag can be reduced, and wearing comfort can be improved.

Therefore, the thickness of the airbag wall is appropriately designed, so that a height difference between an expansion height of a middle part of the airbag assembly 6 and an expansion height of a part close to the press-fitting part can be reduced, to increase the effective pressure area, so as to improve accuracy and stability of blood pressure measurement.

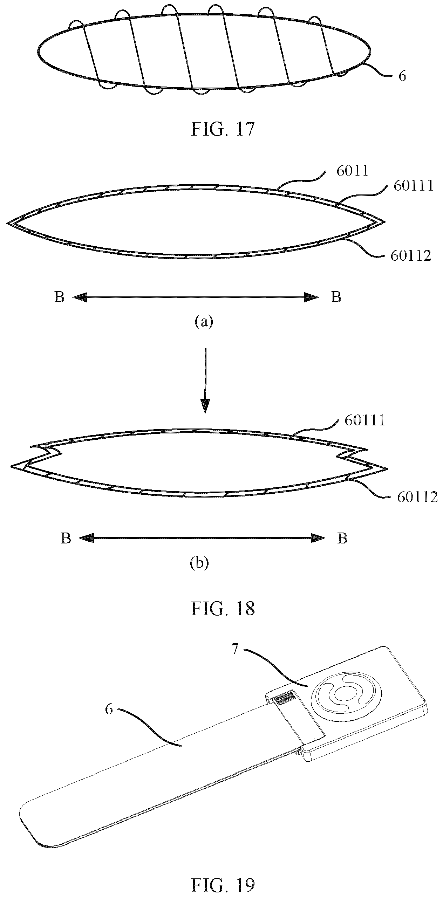

To further improve the shape of the expanded airbag, as shown in FIG. 17, a shape of the expanded airbag assembly 6 may be changed by disposing linear winding on the surface of the airbag assembly 6. The linear winding may be disposed on the surface of the expansion airbag layer 601, or may be disposed on the surface of the protrusion airbag layer 602, or may be disposed on both the surface of the expansion airbag layer 601 and the surface of the protrusion airbag layer 602.

In a possible implementation, a linear winding effect may be implemented by performing winding on the surface of the airbag assembly 6 based on a linear material (for example, a rope or an adhesive tape). In another possible implementation, the linear winding effect may alternatively be implemented by disposing a reinforcing rib inside and/or outside the airbag wall of the airbag assembly 6 in a linear winding manner. This is not limited in embodiments of this application, provided that expansion of a linear winding part is suppressed to some extent when the airbag is expanded.

To further improve the shape of the expanded airbag, the airbag wall 6011 of the expansion airbag layer 601 may be further designed. As shown in (a) in FIG. 18, the airbag wall 6011 of the expansion airbag layer 601 includes a first airbag wall 60111 and a second airbag wall 60112. The first airbag wall 60111 is an airbag wall close to the protrusion airbag layer 602, and the second airbag wall 60112 is an airbag wall away from the protrusion airbag layer 602. A common airbag wall 6011 is smooth without creases. To further increase the expansion height of the part that is of the expansion airbag layer 601 and that is close to the press-fitting part, as shown in (b) in FIG. 18, a folding structure may be disposed at a location that is of the airbag wall of the expansion airbag layer and that is close to the press-fitting part. In a possible implementation, the folding structure may be formed in a hot-pressed airbag wall manner, or the folding structure may be formed in another possible process manner. This is not limited in embodiments of this application.

An embodiment of this application further provides a wearable device. The wearable device integrates a blood pressure measurement function, and may be configured to measure blood pressure. The wearable device may be an electronic device worn on a wrist, like a watch or a wristband, or may be a cuff-type blood pressure meter (worn on an arm to measure blood pressure) or a finger-type blood pressure meter (worn on a finger to measure blood pressure), or may be another electronic device that measures blood pressure based on an airbag.

As shown in FIG. 19, the wearable device may include a watch body 7, a pressure sensor (not shown in the figure), and the airbag assembly 6 provided in embodiments of this application. The pressure sensor is configured to detect air pressure in the airbag assembly 6, and the pressure sensor is electrically connected to the watch body 7. The watch body 7 includes a display, configured to display a blood pressure measurement result.

In a possible implementation, the watch body 7 and the airbag assembly 6 are detachably connected, so that the airbag assembly 6 can be conveniently disassembled and replaced with another wrist strap, to meet various wearing requirements of a user and improve user experience. In another possible implementation, the wearable device further includes a wrist strap, and the airbag assembly 6 may be disposed in the wrist strap. In another possible implementation, the airbag assembly 6 may alternatively be disposed at a layer that is on a surface of the wrist strap and that is close to a skin surface of the user. During normal wearing, the airbag assembly 6 does not need to be inflated, and the wrist strap may maintain a light and thin status, to ensure wearing comfort of the wearable device. When blood pressure measurement needs to be performed, the airbag assembly 6 may be inflated, to apply effective pressure to the skin surface of the user.

The foregoing descriptions are merely optional embodiments of this disclosure, but are not intended to limit this disclosure. The scope of protection is defined by the claims.

## Claims

1. An airbag assembly for blood pressure measurements, wherein the airbag assembly comprises an expansion airbag layer (601) and a protrusion airbag layer (602), wherein
the protrusion airbag layer (602) is disposed on a surface of the expansion airbag layer (601), and a connected first air passage (603) is disposed between the protrusion airbag layer (602) and the expansion airbag layer (601), and is configured to allow air to circulate between the expansion airbag layer (601) and the protrusion airbag layer (602); and
the protrusion airbag layer (602) comprises a plurality of sub-airbags, the plurality of sub-airbags comprise a first sub-airbag (6021) and a second sub-airbag (6022), the first sub-airbag (6021) is disposed at a location closer to a middle part of the expansion airbag layer (601) than the second sub-airbag (6022), a capacity of an inflation cavity of the first sub-airbag (6021) is less than a capacity of an inflation cavity of the second sub-airbag (6022);
**characterized in that**
the expansion airbag layer (601) comprises a first expansion airbag layer (6014) and a second expansion airbag layer (6015) that are stacked, a connected second air passage (604) is disposed between the first expansion airbag layer (6014) and the second expansion airbag layer (6015), and is configured to allow air to circulate between the first expansion airbag layer (6014) and the second expansion airbag layer (6015), the second air passage (604) is of a bar-shaped structure, a separation rib (6041) is disposed in the second air passage (604), and the separation rib (6041) divides the second air passage (604) into a plurality of sub-air passages (6042).

2. The airbag assembly according to claim **1,** wherein the second air passage (604) is disposed in the middle part of the expansion airbag layer (601), a ratio of a width of the second air passage (604) to a width of the expansion airbag layer (601) is greater than 1:10, the width of the expansion airbag layer (601) is determined based on a length of a short side of the expansion airbag layer, and a difference between a distance from the second air passage (604) to a long side of the expansion airbag layer and a distance from the second air passage (604) to the short side of the expansion airbag layer is within a preset range.

3. The airbag assembly according to claim 1 or 2, wherein a length of the sub-air passage (6042) is less than 30 mm, and a ratio of the length of the sub-air passage (6042) to a length of the separation rib (6041) is greater than 2 and less than 8.

4. The airbag assembly according to any one of claims 1 to 3, wherein the first air passage (603) is set to a bar-shaped structure, a separation **rib** is disposed in the first air passage (603), and the separation rib divides the first air passage into a plurality of sub-air passages.

5. The airbag assembly according to any one of claims 1 to 4, wherein a reinforcing rib (6016) is disposed on an inner wall of the expansion airbag layer (601) or an inner wall of the protrusion airbag layer (602).

6. The airbag assembly according to claim 5, wherein a reinforcing rib (6016) is disposed in a middle part of the inner wall of the expansion airbag layer (601) or a middle part of an inner wall of at least one of the sub-airbags.

7. The airbag assembly according to any one of claims 1 to 6, wherein a thickness of an airbag wall of the protrusion airbag layer (602) is less than a thickness of an airbag wall of the expansion airbag layer (601).

8. The airbag assembly according to any one of claims 1 to 7, wherein a thickness of an airbag wall that is of the expansion airbag layer (601) and that is close to the protrusion airbag layer (602) is less than a thickness of an airbag wall that is of the expansion airbag layer (601) and that is away from the protrusion airbag layer (602).

9. The airbag assembly according to any one of claims 1 to 8, wherein the thickness of the airbag wall of the expansion airbag layer (601) or the thickness of the airbag wall of the protrusion airbag layer (602) is uneven.

10. The airbag assembly according to claim 9, wherein
a thickness of an airbag wall close to the middle part of the expansion airbag layer (601) is greater than a thickness of an airbag wall away from the middle part of the expansion airbag layer (601); or
a thickness of an airbag wall of the first sub-airbag (6021) is greater than a thickness of an airbag wall of the second sub-airbag (6022); or
the plurality of sub-airbags comprise a third sub-airbag, and a thickness of an airbag wall close to a middle part of the third sub-airbag is greater than a thickness of an airbag wall away from the middle part of the third sub-airbag.

11. The airbag assembly according to any one of claims 1 to 10, wherein capacities of inflation cavities of the plurality of sub-airbags gradually increase in a direction from a location close to the middle part of the expansion airbag layer (601) to a location away from the middle part of the expansion airbag layer (601).

12. The airbag assembly according to any one of claims 1 to 11, wherein a folding structure is disposed at a location that is on the airbag wall of the expansion airbag layer (601) and that is close to a press-fitting part.

13. The airbag assembly according to any one of claims 1 to 12, wherein
the plurality of sub-airbags comprise a fourth sub-airbag and a fifth sub-airbag that are adjacent to each other, and a connected air passage is disposed between the fourth sub-airbag and the fifth sub-airbag, and is configured to allow air to circulate between the fourth sub-airbag and the fifth sub-airbag; and
the plurality of sub-airbags further comprise a sixth sub-airbag adjacent to the fourth sub-airbag, and no connected air passage is disposed between the fourth sub-airbag and the sixth sub-airbag.

14. The airbag assembly according to any one of claims 1 to 13,
wherein the sub-airbag is of a bar-shaped structure, a rectangular structure, a rhombic structure, or a circular structure; and
wherein an air inlet (6012) is disposed on the expansion airbag layer (601), and the air inlet (6012) allows communication between an inflation cavity (6013) of the expansion airbag layer (601) and external air.

15. A wearable device, wherein the wearable device comprises a watch body (7), a pressure sensor, and the airbag assembly (6) according to any one of claims 1 to 14, wherein
the pressure sensor is configured to detect air pressure in the airbag assembly (6), and the pressure sensor is electrically connected to the watch body (7).

## Patentansprüche

1. Luftkissenanordnung für Blutdruckmessungen, wobei die Luftkissenanordnung eine Aufweitungsluftkissenschicht (601) und eine Vorwölbungsluftkissenschicht (602) umfasst, wobei
die Vorwölbungsluftkissenschicht (602) auf einer Oberfläche der Aufweitungsluftkissenschicht (601) angeordnet ist und ein angeschlossener erster Luftdurchlass (603) zwischen der Vorwölbungsluftkissenschicht (602) und der Aufweitungsluftkissenschicht (601) angeordnet ist und dafür gestaltet ist, das Zirkulieren von Luft zwischen der Aufweitungsluftkissenschicht (601) und der Vorwölbungsluftkissenschicht (602) zu ermöglichen; und
die Vorwölbungsluftkissenschicht (602) mehrere Teilluftkissen umfasst, wobei die mehreren Teilluftkissen ein erstes Teilluftkissen (6021) und ein zweites Teilluftkissen (6022) umfassen, das erste Teilluftkissen (6021) an einer Stelle näher zu einem mittleren Teil der Aufweitungsluftkissenschicht (601) als das zweite Teilluftkissen (6022) angeordnet ist, eine Kapazität eines Aufblashohlraums des ersten Teilluftkissens (6021) geringer als eine Kapazität eines Aufblashohlraums des zweiten Teilluftkissens (6022) ist;
**dadurch gekennzeichnet, dass**
die Aufweitungsluftkissenschicht (601) eine erste Aufweitungsluftkissenschicht (6014) und eine zweite Aufweitungsluftkissenschicht (6015) umfasst, die übereinandergelegt sind, ein angeschlossener zweiter Luftdurchlass (604) zwischen der ersten Aufweitungsluftkissenschicht (6014) und der zweiten Aufweitungsluftkissenschicht (6015) angeordnet ist und dafür gestaltet ist, das Zirkulieren von Luft zwischen der ersten Aufweitungsluftkissenschicht (6014) und der zweiten Aufweitungsluftkissenschicht (6015) zu ermöglichen, der zweite Luftdurchlass (604) eine stabförmige Struktur aufweist, eine Trennrippe (6041) in dem zweiten Luftdurchlass (604) angeordnet ist und die Trennrippe (6041) den zweiten Luftdurchlass (604) in mehrere Teilluftdurchlässe (6042) teilt.

2. Luftkissenanordnung nach Anspruch 1, wobei der zweite Luftdurchlass (604) in dem mittleren Teil der Aufweitungsluftkissenschicht (601) angeordnet ist, ein Verhältnis einer Breite des zweiten Luftdurchlasses (604) zu einer Breite der Aufweitungsluftkissenschicht (601) größer als 1:10 ist, die Breite der Aufweitungsluftkissenschicht (601) basierend auf einer Länge einer kurzen Seite der Aufweitungsluftkissenschicht bestimmt ist und eine Differenz zwischen einem Abstand von dem zweiten Luftdurchlass (604) zu einer langen Seite der Aufweitungsluftkissenschicht und einem Abstand von dem zweiten Luftdurchlass (604) zu der kurzen Seite der Aufweitungsluftkissenschicht innerhalb eines voreingestellten Bereichs liegt.

3. Luftkissenanordnung nach Anspruch 1 oder 2, wobei eine Länge des Teilluftdurchlasses (6042) kleiner als 30 mm ist und ein Verhältnis der Länge des Teilluftdurchlasses (6042) zu einer Länge der Trennrippe (6041) größer als 2 und kleiner als 8 ist.

4. Luftkissenanordnung nach einem der Ansprüche 1 bis 3, wobei der erste Luftdurchlass (603) auf eine stabförmige Struktur festgelegt ist, die Trennrippe in dem ersten Luftdurchlass (603) angeordnet ist und die Trennrippe den ersten Luftdurchlass in mehrere Teilluftdurchlässe teilt.

5. Luftkissenanordnung nach einem der Ansprüche 1 bis 4, wobei eine Verstärkungsrippe (6016) an einer Innenwand der Aufweitungsluftkissenschicht (601) oder einer Innenwand der Vorwölbungsluftkissenschicht (602) angeordnet ist.

6. Luftkissenanordnung nach Anspruch 5, wobei eine Verstärkungsrippe (6016) in einem mittleren Teil der Innenwand der Aufweitungsluftkissenschicht (601) oder einem mittleren Teil einer Innenwand mindestens eines der Teilluftkissen angeordnet ist.

7. Luftkissenanordnung nach einem der Ansprüche 1 bis 6, wobei eine Dicke einer Luftkissenwand der Vorwölbungsluftkissenschicht (602) kleiner als eine Dicke einer Luftkissenwand der Aufweitungsluftkissenschicht (601) ist.

8. Luftkissenanordnung nach einem der Ansprüche 1 bis 7, wobei eine Dicke einer Luftkissenwand, die zur Aufweitungsluftkissenschicht (601) gehört und die nahe der Vorwölbungsluftkissenschicht (602) liegt, kleiner als eine Dicke einer Luftkissenwand ist, die zu der Aufweitungsluftkissenschicht (601) gehört und die fern der Vorwölbungsluftkissenschicht (602) liegt.

9. Luftkissenanordnung nach einem der Ansprüche 1 bis 8, wobei die Dicke der Luftkissenwand der Aufweitungsluftkissenschicht (601) oder die Dicke der Luftkissenwand der Vorwölbungsluftkissenschicht (602) ungleichmäßig ist.

10. Luftkissenanordnung nach Anspruch 9, wobei
eine Dicke einer Luftkissenwand nahe dem mittleren Teil der Aufweitungsluftkissenschicht (601) größer als eine Dicke einer Luftkissenwand fern des mittleren Teils der Aufweitungsluftkissenschicht (601) ist; oder
eine Dicke einer Luftkissenwand des ersten Teilluftkissens (6021) größer als eine Dicke einer Luftkissenwand des zweiten Teilluftkissens (6022) ist; oder
die mehreren Teilluftkissen ein drittes Teilluftkissen umfassen und eine Dicke einer Luftkissenwand nahe einem mittleren Teil des dritten Teilluftkissens größer als eine Dicke einer Luftkissenwand fern des mittleren Teils des dritten Teilluftkissens ist.

11. Luftkissenanordnung nach einem der Ansprüche 1 bis 10, wobei die Kapazitäten von Aufblashohlräumen der mehreren Teilluftkissen in einer Richtung von einer Stelle nahe dem mittleren Teil der Aufweitungsluftkissenschicht (601) zu einer Stelle fern des mittleren Teils der Aufweitungsluftkissenschicht (601) schrittweise zunehmen.

12. Luftkissenanordnung nach einem der Ansprüche 1 bis 11, wobei eine Faltstruktur an einer Stelle angeordnet ist, die sich an der Luftkissenwand der Aufweitungsluftkissenschicht (601) befindet und die nahe einem Presspassungsteil liegt.

13. Luftkissenanordnung nach einem der Ansprüche 1 bis 12, wobei
die mehreren Teilluftkissen ein viertes Teilluftkissen und ein fünftes Teilluftkissen umfassen, die nebeneinander liegen und ein angeschlossener Luftdurchlass zwischen dem vierten Teilluftkissen und dem fünften Teilluftkissen angeordnet ist und dafür gestaltet ist, das Zirkulieren von Luft zwischen dem vierten Teilluftkissen und dem fünften Teilluftkissen zu ermöglichen; und
die mehreren Teilluftkissen ferner ein sechstes Teilluftkissen neben dem vierten Teilluftkissen umfassen und kein Luftdurchlass zwischen dem vierten Teilluftkissen und dem sechsten Teilluftkissen angeordnet ist.

14. Luftkissenanordnung nach einem der Ansprüche 1 bis 13,
wobei das Teilluftkissen eine stabförmige Struktur, eine rechteckige Struktur, eine rhombische Struktur oder eine kreisförmige Struktur aufweist; und
wobei ein Lufteinlass (6012) an der Aufweitungsluftkissenschicht (601) angeordnet ist und der Lufteinlass (6012) eine Verbindung zwischen einem Aufblashohlraum (6013) der Aufweitungsluftkissenschicht (601) und Außenluft ermöglicht.

15. Tragbares Gerät, wobei das tragbare Gerät einen Uhrenkörper (7), einen Drucksensor und die Luftkissenanordnung (6) nach einem der Ansprüche 1 bis 14 umfasst, wobei der Drucksensor dafür gestaltet ist, einen Luftdruck in der Luftkissenanordnung (6) zu erkennen, und der Drucksensor elektrisch mit dem Uhrenkörper (7) verbunden ist.

## Revendications

1. Ensemble de poche gonflable pour effectuer des mesures de pression artérielle, l'ensemble de poche gonflable comprenant une couche de dilatation de poche gonflable (601) et une couche en saillie de poche gonflable (602), dans lequel
la couche en saillie de poche gonflable (602) est agencée sur une surface de la couche de dilatation de poche gonflable (601), et un premier passage d'air communicant (603) est agencé entre la couche en saillie de poche gonflable (602) et la couche de dilatation de poche gonflable (601) et est configuré pour permettre une circulation d'air entre la couche de dilatation de poche gonflable (601) et la couche en saillie de poche gonflable (602) ; et
la couche en saillie de poche gonflable (602) comprend une pluralité de sous-poches gonflables, la pluralité de sous-poches gonflables comprend une première sous-poche gonflable (6021) et une deuxième sous-poche gonflable (6022), la première sous-poche gonflable (6021) est agencée à un emplacement plus proche d'une partie médiane de la couche de dilatation de poche gonflable (601) que la deuxième sous-poche gonflable (6022), une capacité d'une cavité de gonflage de la première sous-poche gonflable (6021) est inférieure à une capacité d'une cavité de gonflage de la deuxième sous-poche gonflable (6022) ;
**caractérisé en ce que**
la couche de dilatation de poche gonflable (601) comprend une première couche de dilatation de poche gonflable (6014) et une deuxième couche de dilatation de poche gonflable (6015) qui sont empilées, un deuxième passage d'air communicant (604) est agencé entre la première couche de dilatation de poche gonflable (6014) et la deuxième couche de dilatation de poche gonflable (6015) et est configuré pour permettre une circulation d'air entre la première couche de dilatation de poche gonflable (6014) et la deuxième couche de dilatation de poche gonflable (6015), le deuxième passage d'air (604) présente une structure en forme de barre, une nervure de séparation (6041) est agencée dans le deuxième passage d'air (604), et la nervure de séparation (6041) divise le deuxième passage d'air (604) en une pluralité de sous-passages d'air (6042).

2. Ensemble de poche gonflable selon la revendication 1, dans lequel le deuxième passage d'air (604) est agencé dans la partie médiane de la couche de dilatation de poche gonflable (601), un rapport d'une largeur du deuxième passage d'air (604) sur une largeur de la couche de dilatation de poche gonflable (601) est supérieur à 1:10, la largeur de la couche de dilatation de poche gonflable (601) est déterminée sur la base d'une longueur d'un petit côté de la couche de dilatation de poche gonflable, et une différence entre une distance séparant le deuxième passage d'air (604) d'un grand côté de la couche de dilatation de poche gonflable et une distance séparant le deuxième passage d'air (604) du petit côté de la couche de dilatation de poche gonflable s'inscrit dans une plage prédéfinie.

3. Ensemble de poche gonflable selon la revendication 1 ou 2, dans lequel une longueur du sous-passage d'air (6042) est inférieure à 30 mm, et un rapport de la longueur du sous-passage d'air (6042) sur une longueur de la nervure de séparation (6041) est supérieur à 2 et inférieur à 8.

4. Ensemble de poche gonflable selon l'une quelconque des revendications 1 à 3, dans lequel le premier passage d'air (603) présente une structure en forme de barre, une nervure de séparation est agencée dans le premier passage d'air (603), et la nervure de séparation divise le premier passage d'air en une pluralité de sous-passages d'air.

5. Ensemble de poche gonflable selon l'une quelconque des revendications 1 à 4, dans lequel une nervure de renfort (6016) est agencée sur une paroi intérieure de la couche de dilatation de poche gonflable (601) ou sur une paroi intérieure de la couche en saillie de poche gonflable (602).

6. Ensemble de poche gonflable selon la revendication 5, dans lequel une nervure de renfort (6016) est agencée dans une partie médiane de la paroi intérieure de la couche de dilatation de poche gonflable (601) ou dans une partie médiane d'une paroi intérieure d'au moins une des sous-poches gonflables.

7. Ensemble de poche gonflable selon l'une quelconque des revendications 1 à 6, dans lequel une épaisseur d'une paroi de poche gonflable de la couche en saillie de poche gonflable (602) est inférieure à une épaisseur d'une paroi de poche gonflable de la couche de dilatation de poche gonflable (601).

8. Ensemble de poche gonflable selon l'une quelconque des revendications 1 à 7, dans lequel une épaisseur d'une paroi de poche gonflable de la couche de dilatation de poche gonflable (601) qui est proche de la couche en saillie de poche gonflable (602) est inférieure à une épaisseur d'une paroi de poche gonflable de la couche de dilatation de poche gonflable (601) qui est éloignée de la couche en saillie de poche gonflable (602).

9. Ensemble de poche gonflable selon l'une quelconque des revendications 1 à 8, dans lequel l'épaisseur de la paroi de poche gonflable de la couche de dilatation de poche gonflable (601) ou l'épaisseur de la paroi de poche gonflable de la couche en saillie de poche gonflable (602) est non uniforme.

10. Ensemble de poche gonflable selon la revendication 9, dans lequel une épaisseur d'une paroi de poche gonflable proche de la partie médiane de la couche de dilatation de poche gonflable (601) est supérieure à une épaisseur d'une paroi de poche gonflable éloignée de la partie médiane de la couche de dilatation de poche gonflable (601) ; ou
une épaisseur d'une paroi de poche gonflable de la première sous-poche gonflable (6021) est supérieure à une épaisseur d'une paroi de poche gonflable de la deuxième sous-poche gonflable (6022) ; ou
la pluralité de sous-poches gonflables comprend une troisième sous-poche gonflable, et une épaisseur d'une paroi de poche gonflable proche d'une partie médiane de la troisième sous-poche gonflable est supérieure à une épaisseur d'une paroi de poche gonflable éloignée de la partie médiane de la troisième sous-poche gonflable.

11. Ensemble de poche gonflable selon l'une quelconque des revendications 1 à 10, dans lequel des capacités de cavités de gonflage de la pluralité de sous-poches gonflables augmentent progressivement dans une direction allant d'un emplacement proche de la partie médiane de la couche de dilatation de poche gonflable (601) vers un emplacement éloigné de la partie médiane de la couche de dilatation de poche gonflable (601).

12. Ensemble de poche gonflable selon l'une quelconque des revendications 1 à 11, dans lequel une structure de pliage est agencée à un emplacement sur la paroi de poche gonflable de la couche de dilatation de poche gonflable (601) qui est proche d'une partie d'assemblage par pression.

13. Ensemble de poche gonflable selon l'une quelconque des revendications 1 à 12, dans lequel
la pluralité de sous-poches gonflables comprend une quatrième sous-poche gonflable et une cinquième sous-poche gonflable adjacentes l'une à l'autre, et un passage d'air communicant est agencé entre la quatrième sous-poche gonflable et la cinquième sous-poche gonflable et est configuré pour permettre une circulation d'air entre la quatrième sous-poche gonflable et la cinquième sous-poche gonflable ; et
la pluralité de sous-poches gonflables comprend en outre une sixième sous-poche gonflable adjacente à la quatrième sous-poche gonflable, et aucun passage d'air communicant n'est agencé entre la quatrième sous-poche gonflable et la sixième sous-poche gonflable.

14. Ensemble de poche gonflable selon l'une quelconque des revendications 1 à 13, dans lequel la sous-poche gonflable présente une structure en forme de barre, une structure rectangulaire, une structure en losange, ou une structure circulaire ; et
dans lequel une entrée d'air (6012) est agencée sur la couche de dilatation de poche gonflable (601), et l'entrée d'air (6012) permet une communication entre une cavité de gonflage (6013) de la couche de dilatation de poche gonflable (601) et l'air extérieur.

15. Dispositif à porter sur soi, le dispositif à porter sur soi comprenant un corps de montre (7), un capteur de pression, et l'ensemble de poche gonflable (6) selon l'une quelconque des revendications 1 à 14, dans lequel
le capteur de pression est configuré pour détecter une pression d'air dans l'ensemble de poche gonflable (6), et le capteur de pression est électriquement relié au corps de montre (7).
